# EUROPEAN PATENT SPECIFICATION

(11) **EP 4 556 907 B1**
(45) Date of publication and mention of the grant of the patent: **04.02.2026**
(21) Application number: 23210793.8
(22) Date of filing: 20.11.2023
(51) Int. Cl.: G01N 33/532, G01N 33/68

(54) **SITE-SPECIFIC CONJUGATION OF SINGLE DOMAIN ANTIBODY FRAGMENTS (VHH) USING EQUILIBRIUM TRANSFER ALKYLATION REAGENT (ETAC)**
ORTSSPEZIFISCHE KONJUGATION VON EINZELDOMÄNENANTIKÖRPERFRAGMENTEN (VHH) UNTER VERWENDUNG EINES GLEICHGEWICHTSTRANSFERALKYLIERUNGSREAGENZ (ETAC)
CONJUGAISON SPÉCIFIQUE À UN SITE DE FRAGMENTS D'ANTICORPS À DOMAINE UNIQUE (VHH) À L'AIDE D'UN RÉACTIF D'ALKYLATION PAR TRANSFERT D'ÉQUILIBRE (ETAC)

(43) Date of publication of application: 21.05.2025
(73) Proprietor: Miltenyi Biotec B.V. & Co. KG, 51429 Bergisch Gladbach (DE)
(72) Inventor: WERSCHAU, Niels, 51429 Bergisch Gladbach (DE); WAGNER, Michael, 51429 Bergisch Gladbach (DE); MITTELSTÄT, Jörg, 51429 Bergisch Gladbach (DE); FAUERBACH, Jonathan, 51429 Bergisch Gladbach (DE)
(74) Representative: Kisters, Michael Marcus

(56) References cited:
- EP-A1- 3 620 464
- EP-A1- 4 234 559
- WO-A1-2009/047500
- US-A1- 2016 000 933
- KAROLINA PECIAK: "Site-selective protein conjugation at histidine", CHEMICAL SCIENCE, vol. 10, no. 2, 2019, pages 427 - 439, XP055709486
- DEL ROSARIO R B ET AL: "SULFHYDRYL SITE-SPECIFIC CROSS-LINKING AND LABELING OF MONOCLONAL ANTIBODIES BY A FLUORESCENT EQUILIBRIUM TRANSFER ALKYLATION CROSS-LINK REAGENT", BIOCONJUGATE CHEMISTRY, vol. 1, no. 1, 1990, pages 51 - 59, XP002313938
- YUEHUA CONG ET AL: "Site-Specific PEGylation at Histidine Tags", BIOCONJUGATE CHEMISTRY, vol. 23, no. 2, 2012, pages 248 - 263, XP055074953

## Description

### BACKGROUND

Antibodies and antibody fragments thereof e.g. VHHs, are an important class of biomolecules for immunotherapy. Due to their unique molecular properties e.g. small size, simple folding, and improved tissue penetration, VHHs are becoming more important towards new therapeutic approaches. One potential novel application for VHHs, is their use as adapter molecules in combination with AdCAR^{™} T cells.

Therefore, VHHs need to be modified or tagged for such purposes. One approach is chemical conjugation to a chemical tag, e.g. biotin, thiamine, peptides, DBCO, oligonucleotides, peptide nucleic acids (PNAs) to name a few, to get recognized by the AdCAR^{™} T cells in order to crosslink tumor cells and CAR T cells for tumor cell killing. The use of adapter molecules in conjunction with AdCAR^{™} T cells is the ability to control dosing, and the consequential safety as in case it is needed, it can be easily removed featuring minimal risks to patients. To be suitable as pharmaceutical, adapter molecules' chemical conjugation must be site-specific and result in a homogenous and well-defined product that is stable under physiological conditions after injection.

One opportunity to enable site-specific conjugation is the incorporation of engineered cysteines. However, free cysteines can form disulfide bonds leading to the aggregation of the protein during production or purification. Another strategy that is used currently is maleimide-based conjugation of free cysteines. The limitation of this approach is that maleimide thiol adducts can generate labile chemical conjugation under physiologically reducing conditions. The occurring retro Michal-addition would lead to adapter Molecules that are non-functional and potential block epitopes for conjugated adapter molecules [1,2].

There are many known methods for the site-specific conjugation of single domain antibody fragments, e.g. maleimide addition to engineered cysteine or Sortase reactions [3].

For example, US11021544B2 discloses the use of the ETAC system (Thiobridging) to dimerize VHHs, wherein VHHs featuring a C-terminal extension comprising a cysteine moiety are linked via the ETAC system (Thiobridging) to yield chemically linked Nanobody dimers introducing a diagnostic, therapeutic or labelling agent.

Further, publication [5] discloses that conjugation by bis-alkylation using reagents can be equilibrium controlled *via* an addition-elimination reaction that is reliant on the Michael reaction. Covalent conjugation occurs with 2 amino acids close in space (*e.g.* the two cysteine thiols from a disulfide or two histidines in a C- or N-terminal his-tag).

In publication [6], the modification of His tags by ETAC reagents has been documented for the possibility to give rise to multiple additions of the reagents. The modification of the His tag is not stable against strong Michael donors like DTT. To stabilize the conjugate, a reduction of the introduced ketone in the ETAC linker is achieved using triacetoxy borohydride which might not be compatible with the stability of the protein.

Accordingly, there is a need for a conjugation method is needed that is site-specific, stable under physiological conditions, and not prone to protein aggregation as seen with disulfide bonds.

### OBJECT OF THE INVENTION

Here were could demonstrate that the ETAC-biotin linker can specifically monolabel single VHHs units by linking one terminal cysteine with a histidine residue that is in direct proximity to the terminal cysteine, thus targeting a cysteine-histidine motif. By providing an excess of ETAC-biotin linker the ETAC linker reacted with multiple Michael acceptors within the VHH. We were able to remove ETAC-biotin labels crosslinking single histidine residue under the addition of competing nucleophilic agent (e.g. TCEP) leading to an enrichment of single-site mono-specific conjugated VHHs. No further processing was needed to remove the non-conjugated product (confirmed by LC-MS experiments). To the best of our knowledge, a double Michael addition reaction targeting one cysteine and one neighboring histidine in the same sequence followed by selective enrichment of site-specific single-label VHHs via elimination of multi-label targets using a nucleophile such as TCEP is a unique approach and has not been yet reported.

Object of the invention is a method according to claim 1 for providing a polypetide with a detectable label, wherein the polypetide comprises at least 5 amino acids of which a first amino acid is C (Cysteine) and at least one second amino acid is selected from the group consisting of S (Serine) , T (Threonine) , Y (Tyrosine), K (Lysine), H (Histidine) and R (Arginine) **characterized by** the steps
a) initiating Michael addition between a first and a second amino acid by providing a reagent according to formula (I) 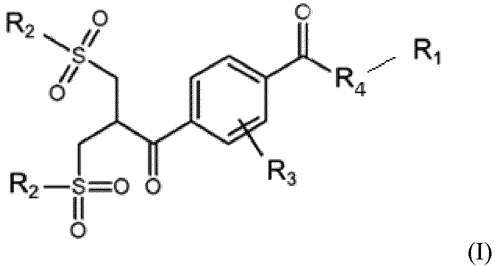 With
   R1 = -Biotin, Thiamine, Peptide with 2 - 30 amino acids, oligonucleotides with 6 - 100 nucleotides, -DBCO, DBCO, -N3,N3 and -Fluorophores for example FITC, Cy dye, Vio dye), -Fluorescent protein like GFP, RFP
   R2 = substituted or unsubstituted aromatic residues like toluene or phenyl,
   R3 = H, F, NO2, alkyl with 1 to 5 carbon atoms
   R4 = direct bond, substituted or unsubstituted alkyl, amin or amid residue with 1 to 20 carbon atom like (-CH2-CH2-CH2-CH2-CH2-CH2-) (LC), or poly ethylene glycole (PEG)
b) Adding a nucleophilic agent to remove the Michael addition products between two second amino acid by retro-Michael addition.

The term "polypeptide" refers to any molecule comprised of a chain of at least 5 amino acids. This includes molecules ranging from small peptides, which are polypeptides with a molecular weight from 300 Da to 7 kDa, to larger forms such as single-domain antibodies (VHHs) that have a molecular weight between 8-15 kDa, antibody fragments typically in the range of 20-50 kDa, and full-sized antibodies like the IgG species, which have a molecular weight of approximately 140-160 kDa.

ETAC-biotin-linked VHHs showed specific binding to their respective tumor antigens indicating correct folding and function. Furthermore, ETAC-biotin conjugated VHHs induced tumor cell lysis and cytokine secretion of biotin-specific adapter CAR.

Compared to a single attachment site, e.g. via a maleimide, the bridged modification of the VHH might provide better stability against de-conjugation and higher rigidity of the introduced labeling agent possibly enhancing binding to a secondary reagent (e.g. CAR T cell).

### BRIEF DESCRIPTION OF THE DRAWINGS

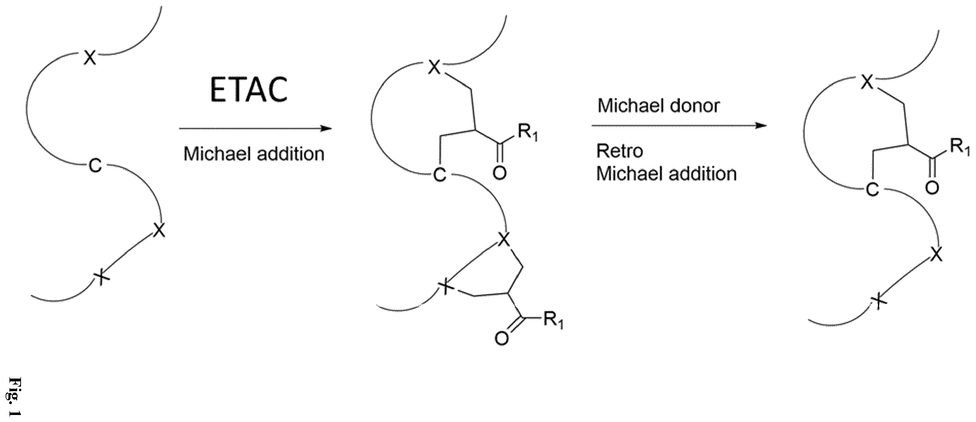

Fig. 1 shows the general concept of site-specific conjugation of polypeptides using the ETAC reagent.

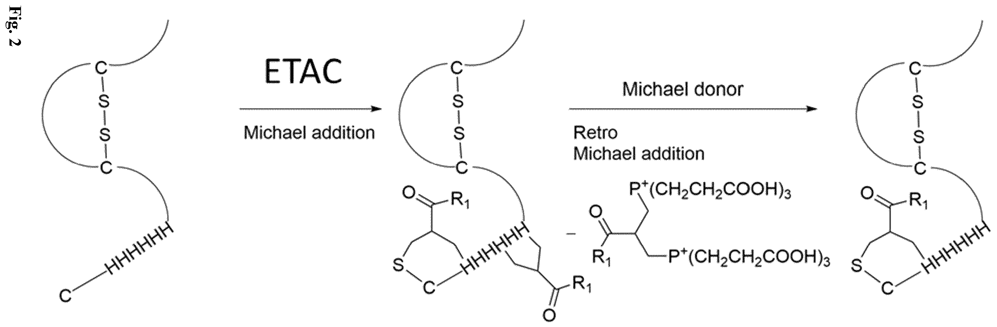

Fig. 2 shows the general concept of site-specific conjugation of single domain antibodies using the ETAC reagent.
Fig. 3 shown a ESI-MS spectrum of ETAC-biotin labeled EGFR specific VHHs before and after Retro Michael addition according to example 1.1
Fig 4 shows a peptide mass finger print (PMF) of ETAC-biotin labeled EGFR specific VHHs indicating the site of conjugation according to example 1.2
Fig. 5 shows the binding of ETAC-biotin labeled EGFR specific VHHs on antigen positive cells according to example 1.3
Fig. 6 shows specific killing of EGFR positive tumor cells via biotin-specific adapter CAR T cells in presence of ETAC-biotin labeled EGFR specific VHH adapter molecules according to example 1.4
Fig. 7 shows ESI-MS spectrum of ETAC-biotin labeled CD56 specific VHHs before and after Retro Michael addition according to example 2.1 and 2.2
Fig. 8 shows the binding of ETAC-biotin labeled CD56 specific VHHs on antigen positive cells or antigen negative cells according to example 2.3

### DETAILED DESCRIPTION

A polypeptide with at least one a reduced Cysteine (C) and at least one nucleophilic amino acid (X) is treated with the ETAC reagent. The ETAC compound reacts in a Michael addition either with a C and amino acid X or with amino acid X and second amino acid X. By treating this reaction products with a Michael donor e.g. a reducing agent, ETAC conjugated to amino acid X and second amino acid X are removed.

The VHH contains a terminal cysteine and sequence of histidines. To enable the conjugation of the VHH with the ETAC reagent the terminal cysteine is reduced leaving the intramolecular disulfide bridge intact. Next, the ETAC compound reacts in a Michael addition with the terminal reduced cysteine and with one of the histidines. In addition, the ETAC compounds is conjugated to only histidines. By treating this reaction product with a Michael donor e.g. a reducing agent, ETAC compounds crosslinked with only histidines are removed from the VHH. ETAC compounds linked to cysteines and histidine are stable in presence of a Michael donor and remain crosslinked.

In an embodiment of the invention, the at least one second amino acid is provided at the C-terminus of the polypeptide.

The nucleophilic agent may be selected from the group consisting of Dithiothreitol (DTT), Tris(2-carboxyethyl)phosphine (TCEP), Triphenylphosphine (PPh3), Hydrazine (N2H4), Tetrakis(dimethylamino)ethylene (TDAE), Mercaptoethanol, Formic acid, Trichlorosilane (HSiCl3), Phosphine (PH3), Dimethyl sulfide borane (Me2S•BH3), Thioacetic acid, Tris(trimethylsilyl)phosphine, Tri-n-hexylphosphine,, Ammonia (NH3), Cysteine, Selenocysteine, Thiophenole, Glutathione (GSH), Iodide, Methylamine, Hydrogen sulfide

Preferable, the reagent has formula (II)

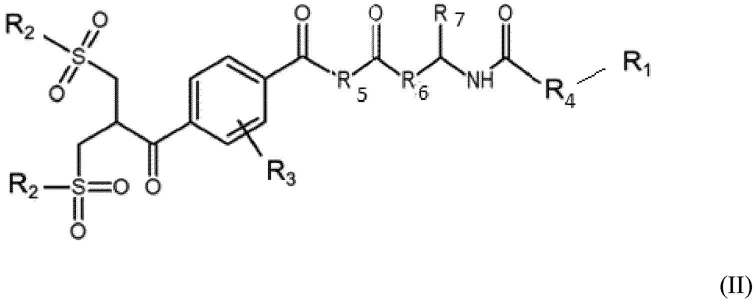

Or formula (III) 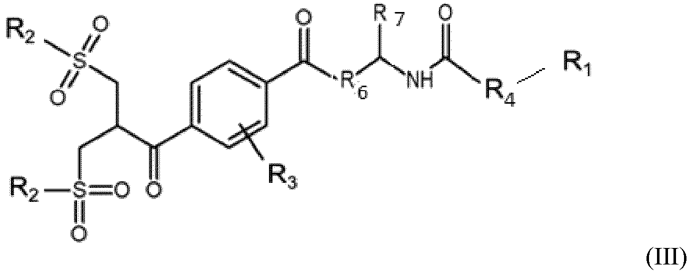 With
R1 = -Biotin, Thiamine, Peptide with 2 - 30 amino acids, oligonucleotides with 6 - 100 nucleotides, -DBCO, DBCO, -N3,N3, -Fluorophore
R2 = substituted or unsubstituted aromatic residues
R3 = H, F, NO2, alkyl with 1 to 5 carbon atoms
R4 = direct bond, substituted or unsubstituted alkyl, amin or amid residue with 1 to 20 carbon atoms
R5 = direct bond, substituted or unsubstituted alkyl or amin residue with 1 to 20 carbon atoms
R6 = direct bond, substituted or unsubstituted alkyl or amin residue with 1 to 20 carbon atoms
R7: substituted or unsubstituted alkyl, carboxy, amin or hydroxy residue with 1 to 20 carbon atoms

Most preferred, the reagent has one of the formulas (IV) to (VII)

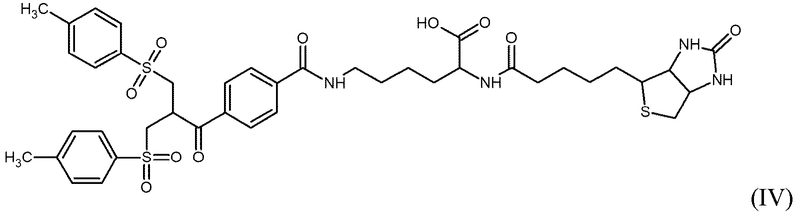

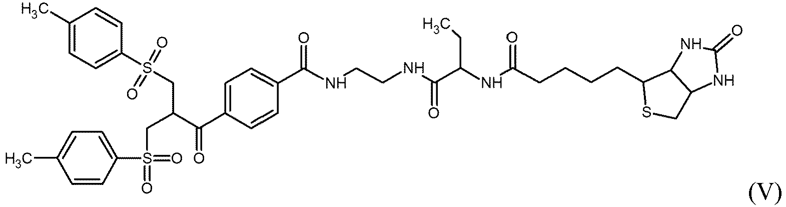

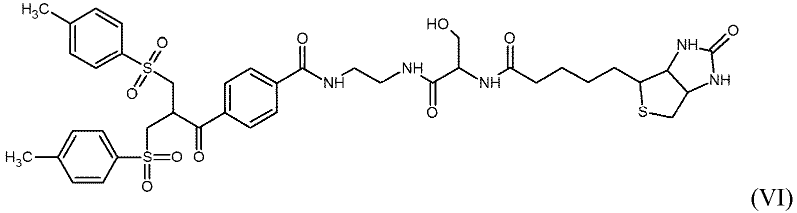

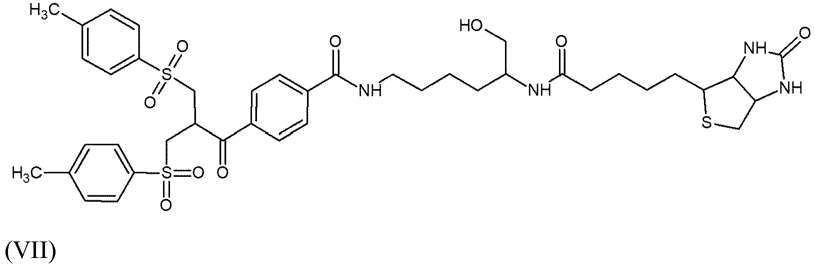

### USE OF THE METHOD

The method of the invention is especially useful to provide polypetides with in CAR-Adapter molecules, in Cell staining reagents, in cell separation/ labeling/ enrichment/ depletion processes, in protein purification and for Cell activation with a detectable label.

### LITERATURE

1. Baldwin, A.D.; Kiick, K.L. Tunable Degradation of Maleimide-Thiol Adducts in Reducing Environments. Bioconjug. Chem. 2011, 22, 1946-1953, doi: 10.1 021/BC200 148V /SUPPL FILE/BC200 148V SI 00 1.PDF.
2. Baldwin, A.D.; Kiick, K.L. Reversible Maleimide-Thiol Adducts Yield Glutathione-Sensitive Poly(Ethylene Glycol)-Heparin Hydrogels. Polym. Chem. 2012, 4, 133-143, doi:10.1039/C2PY20576A.
3. Schumacher, D.; Helma, J.; Schneider, A.F.L.; Leonhardt, H.; Hackenberger, C.P.R. Nanobodies: Chemical Functionalization Strategies and Intracellular Applications. Angew. Chem. Int. Ed. Engl. 2018, 57, 2314, doi:10.1002/ANIE.201708459.
4. Nanobody Dimers Linked via C-Terminally Engineered Cysteins US11021544B2 2021.
5. Peciak, K.; Laurine, E.; Tommasi, R.; Choi, J.W.; Brocchini, S. Site-Selective Protein Conjugation at Histidine. Chem. Sci. 2019, 10, 427-439, doi:10.1039/C8SC03355B.
6. Cong, Y.; Pawlisz, E.; Bryant, P.; Balan, S.; Laurine, E.; Tommasi, R.; Singh, R.; Dubey, S.; Peciak, K.; Bird, M.; et al. Site-Specific PEGylation at Histidine Tags. Bioconjug. Chem. 2012, 23, 248-263, doi:10.1021/BC200530X/SUPPLFILE/BC200530X_SI_001.PDF.

### EXAMPLES

### Example 1 Site-specific conjugation of EGFR specific VHHs with the ETAC-biotin-linker

### 1.1 Conjugation of EGFR specific VHHs with multiple ETAC-biotin-linker

As a model VHH we were using an anti-EGFR specific VHH with a C-terminal histidine tag followed by flexible glycine serin linker and a glycine capped cysteine residue as Michael donors.

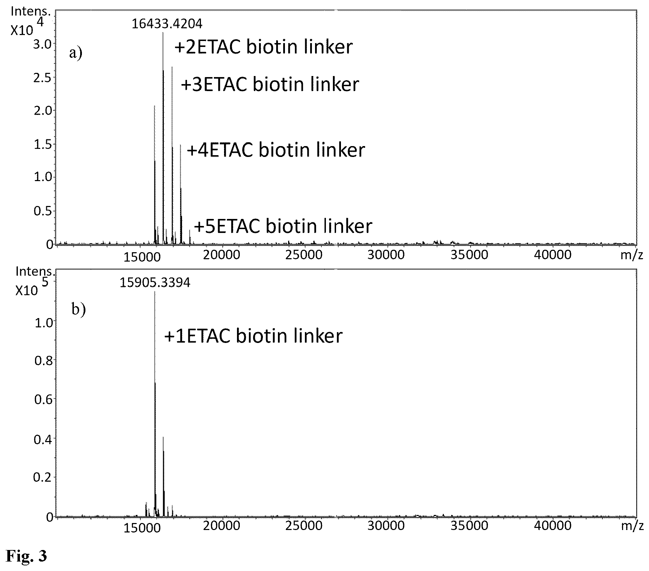

The terminally introduced cysteine of the EGFR VHH was reduced with 3 molar equivalents of TCEP for 1h at 21°C. The reaction was done in PBS buffer with 5 mM EDTA (PE-buffer) at pH 7.8. Next, TCEP was removed via a desalting column contain Sephadex G-25 resin and PE-buffer. Afterwards, the ETAC-biotin labeling molecule was added to the reduced and purified EGFR VHH. The ETAC-biotin labeling molecule was previously dissolved in DMSO and added at 4 molar equivalents to the EGFR VHH. DMSO was added in sufficient amount to the reaction mixture in order to reach a final concentration of 10% DMSO in the final reaction mixture. The conjugation reaction was kept for 2h at 21°C. Next, the reaction mixture was applied to a previously PBS buffer equilibrated molecular weight cut off column (Merck Millipore) and washed with four column volumes of 1X PBS. The protein concentration of the final product was analyzed via absorption at 280 nanometers.
The product of the coupling reaction was analyzed via liquid chromatography coupled to mass spectrometry (Figure 3a). The analytical results indicated that each VHH molecule was bonded to between one and five ETAC-biotin-linker units, with the predominant configuration consisting of two ETAC-biotin-linkers per VHH.

### 1.2 Enrichment of single ETAC-biotin-linker labeled EGFR specific VHHs via retro Michael addition

ETAC-biotin labels that did not react with one cysteine were selectively removed via a retro Michael reaction by adding a nucleophilic reducing agent. Therefore, 250mM TCEP were added per 13µL VHH for 10 min at 37°C. This led to a VHH polypeptide containing a single ETAC-Biotin label with a MW of 15905 Da as confirmed by mass spectrometry (Figure 3b). After the TCEP step, this yields to the final and desired product.

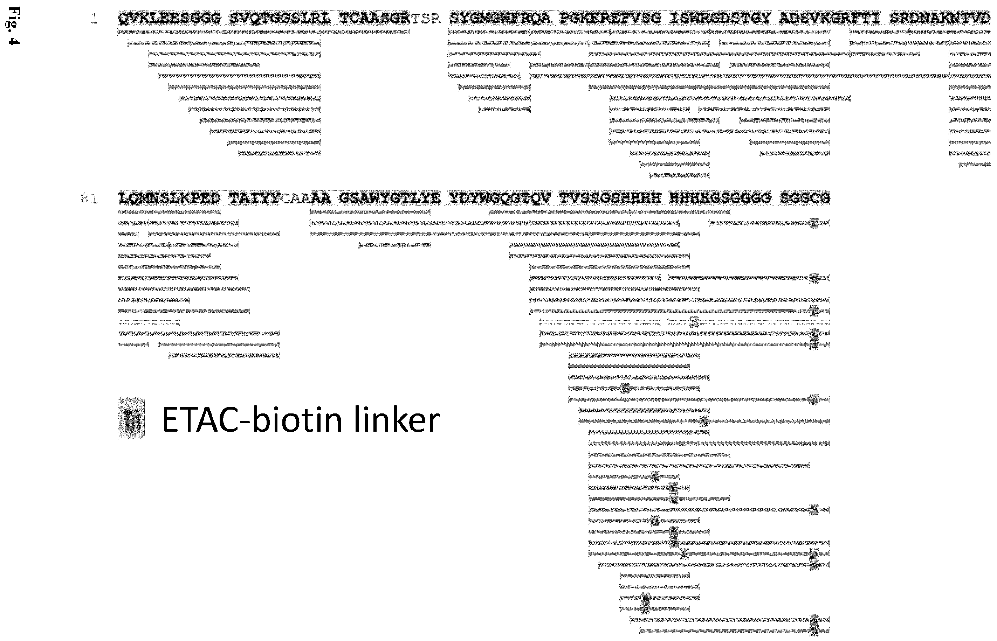

The sites of the conjugation of the ETAC-biotin to the EGFR specific VHH was analyzed via peptide mass fingerprint (PMF) (Figure 4). The PMF showed successful conjugation of the ETAC-biotin label to the cysteine and histidine residues at the carboxy terminus of the VHH. This demonstrates linking of one terminal cysteine with a histidine residue that is in proximity to the terminal cysteine. This describes a unique site-specific labeling site which can be addressed by the ETAC based crosslinkers, providing an alternative motif to cysteine-cysteine.

### 1.3. The use of ETAC-biotin-linker labeled EGFR-specific VHHs in flow cytometry

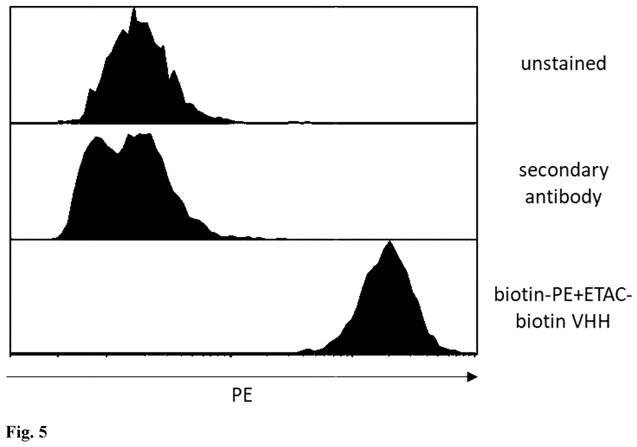

To show the application of ETAC-biotin labeled EGFR-specific VHHs for flow cytometry, EGFR-specific VHH molecules were used to detect tumor cells that are known to express EGFR. Therefor, 10µg/mL of *ETAC-biotin labeled EGFR specific VHHs* were incubated with EGFR positive tumor cells for 1h at 4°C. Cell suspension was washed with PE-buffer containing 0.5% BSA (PEB-buffer) and a secondary staining with a PE labeled biotin specific secondary antibody was done (REA746). Next, the cell suspension was washed with PEB. Binding of ETAC-biotin labeled EGFR specific VHHs to EGFR positive tumor cells and the presence of the ETAC-biotin-linker was detected via secondary staining with a PE labeled biotin-specific secondary antibody. Signal intensity of unstained tumor cells (Figure 5 top row histogram) or on cells stained with only the biotin-specific secondary antibody (Figure 5 middle row histogram) was substantially decreased compared to the EGFR positive tumor cells incubated with ETAC-biotin labeled anti EGFR VHHs and the biotin-specific secondary PE labeled antibody (Figure 5 bottom row histogram). This validates the conjugation of the ETAC-biotin-linker to the EGFR-specific VHH. In addition, the data validate that the conjugation process does not disrupt the functional properties of the EGFR -specific VHH binding its epitope.

### 1.4. The use of ETAC-biotin-linker labeled EGFR-specific VHHs as adapter molecules for adapter CAR T cells

CAR T cells are immune cells that are genetically engineered to express a chimeric antigen receptor (CAR) that does not occur naturally in T cells. This receptor is specific for a certain antigen expressed on the surface of a tumor cell. After engaging the tumor cells the CAR T cell will induce tumor cell lysis. In contrast, an adapter CAR T cell expresses a CAR that is specific for tagged polypeptide and not for an antigen expressed by a tumor cell. This polypeptide e.g. a VHH will mediate the biding between the adapter CAR T cell and the tumor cells. In absence of this adapter no tumor cell lysis will be induced. By using different adapter molecules one CAR T cell can target more than one tumor antigen. This mode of action increases the safety and therapeutic efficacy.

### 1.4.1 Generation of ETAC-biotin-linker specific adapter CAR Tcells

ETAC-biotin CAR T cells contained an biotin specific scFv as binding moiety. The scFv was linked to human CD8 transmembrane domain via hIgG4 hinge domain. The signaling domain was composed of 4-1BB and CD3ζ.

### 1.4.2 Generation of LV particles and titration

Lentiviral vector particles were manufactured via transient transfection of HEK-293T cells. The lentiviral vector particles were pseudotyped with VSV-G. For transfection HEK-293T cells were seeded in T175 culture flasks in DMEM supplemented with 2 mM L-Glutamine and 10 % FCS 3 days prior to transfection. At the day of transfection the culture medium was removed and replaced by DMEM supplemented with 2 mM L-Glutamine. The cells were transfected with a three-plasmid system encoding for VSV-G, gag/pol/rev and the psi positive transfer vector.

After 48h the supernatant was collected and centrifuged for 10 min at 1000 rpm to remove cellular debris. In addition, the supernatant was filtrated trough a 0,45 µm filter. The pellet was re-suspended in ice cold PBS and stored at -80 °C.

### 1.4.3 Transduction, cultivation and analysis of ETAC-biotin-linker specific adapter CAR Tcells

Anti-biotin adapter CAR T cells were manufactured using primary T cells from healthy donors. T cells were isolated from PBMC with the PAN T cell isolation Kit (Miltenyi Biotec) according to the manufactures protocol. Prior to transduction 2E6 T cells were seeded in a 24 well plate with 2 mL TexMACS medium (Miltenyi) supplemented with IL-7 (Miltenyi Biotec), IL-15 (Miltenyi Biotec) and Transact (Miltenyi Biotec).

After 24 h T cells were transduced with an MOI of 5 by adding the corresponding volume of lentiviral vector particles. On day 3 post activation the culture medium was removed and replaced by TexMACS medium (Miltenyi Biotec) supplemented with IL-7 (Miltenyi Biotec), IL-15 (Miltenyi Biotec).

Frequency of anti-biotin adapter CAR positive T cells was analyzed on day 6 after transduction via flow cytometry using a biotinylated PE conjugate (Miltenyi Biotec). Anti-biotin adapter CAR T cells were used for functional assays on day 10 after transduction.

### Example 5 Functionality of EGFR specific VHHs as adapter molecules for ETAC-biotin-linker specific adapter CAR Tcells

To test the application of ETAC biotin conjugated EGFR specific VHHs as adapter molecules for biotin specific anti-biotin adapter CAR T cells, antigen positive tumor cells and ETAC-biotin labeled EGFR specific VHHs were cultured either in presence or absence of biotin-specific adapter CAR T cells. Therefore, 20.000 biotin specific adapter CAR T cells and 5.000 tumor cells were seeded in RPMI with 2 mM L-glutamine and 10% FCS in a 96 well flat bottom plate.

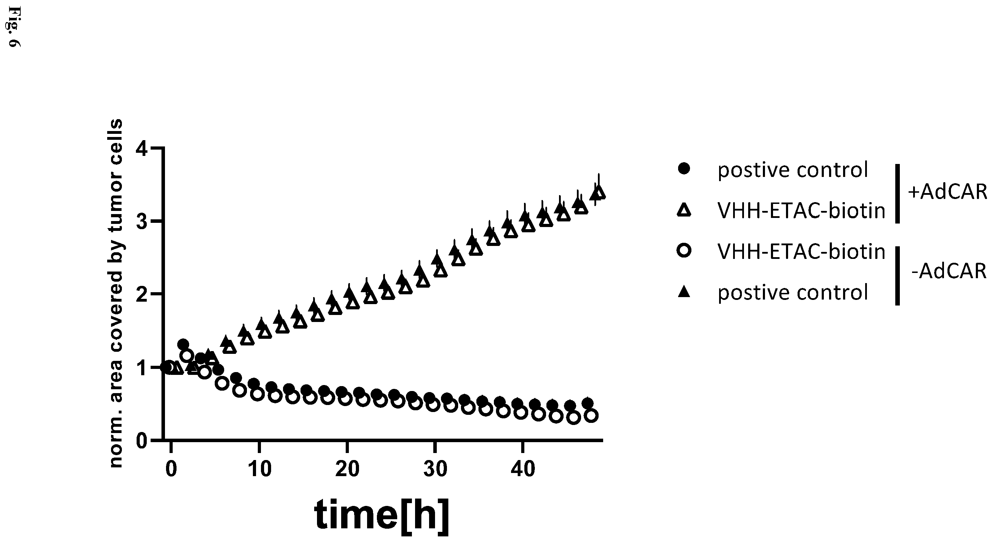

Next, 50 µL ETAC-biotin labeled EGFR specific VHH was added to a final concentration of 5 nM. As a positive control a functional tumor specific adapter molecule was used. The co culture was incubated at 37°C and 5% CO₂. Tumor cell lysis was analyzed via live cell fluorescent microscopy of tumor cells using the IncuCyte^{®} S3 system. Data shown were obtained from (n=3) independent donors and plotted with mean and ± 1 standard deviation (Figure 6).
Only in presence of biotin-specific adapter CAR T cells, the tumor cell lysis was induced. The potency of ETAC-biotin linked VHHs was comparable to a control adapter. These results indicate suitable crosslinking of tumor cells and adapter CAR T cells via ETAC-biotin labeled VHHs.
In summary, this shows that ETAC-biotin-linker labeled EGFR-specific VHHs are functional adapter molecules for biotin-specific adapter CAR T cells.

### Example 2 Site-specific conjugation of CD56 specific VHHs with the ETAC-biotin-linker

As a model VHH we were using an anti-CD56 specific VHH with a C-terminal histidine tag followed by flexible glycine serin linker and a glycine capped cysteine residue as Michael donors.

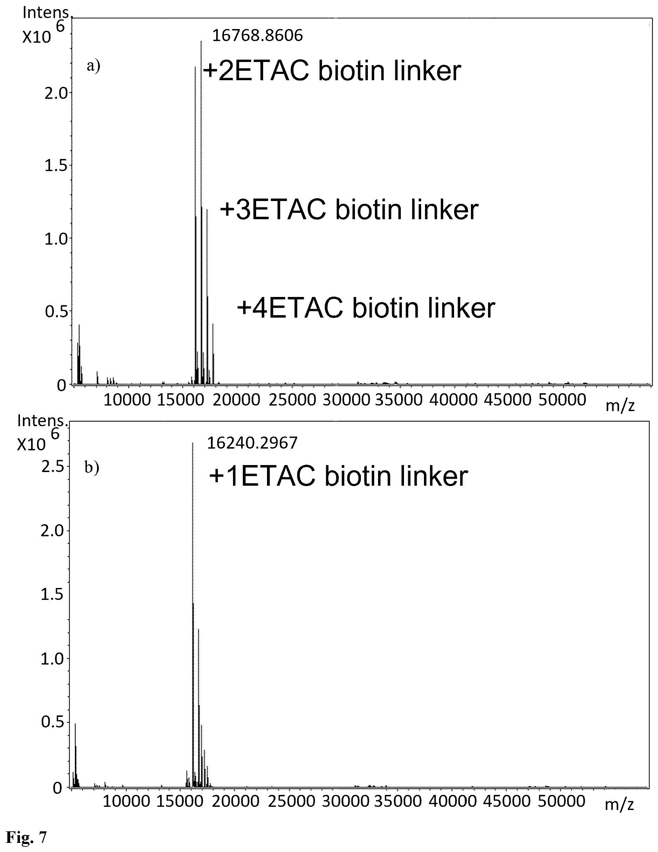

The terminally introduced cysteine of the CD56 VHH was reduced with 3 molar equivalents of TCEP for 1h at 21°C. The reaction was done in PBS buffer with 5 mM EDTA (PE-buffer) at pH 7.8. Next, TCEP was removed via a desalting column contain Sephadex G-25 resin and PE-buffer. Afterwards, the ETAC-biotin labeling molecule was added to the reduced and purified CD56 VHH. The ETAC-biotin labeling molecule was previously dissolved in DMSO and added at 4 molar equivalents to the CD56 VHH. DMSO was added in sufficient amount to the reaction mixture in order to reach a final concentration of 10% DMSO in the final reaction mixture. The conjugation reaction was kept for 2h at 21°C. Next, the reaction mixture was applied to a previously PBS buffer equilibrated molecular weight cut off column (Merck Millipore) and washed with four column volumes of 1X PBS. The protein concentration of the final product was analyzed via absorption at 280 nanometers.
The product of the coupling reaction was analyzed via liquid chromatography coupled to mass spectrometry (Figure 7a). The analytical results indicated that each VHH molecule was bonded to between one and four ETAC-biotin-linker units, with the predominant configuration consisting of two ETAC-biotin-linkers per VHH.

### 2.2 Enrichment of single ETAC-biotin-linker labeled CD56 specific VHHs via retro Michael addition

ETAC-biotin labels that did not react with one cysteine were selectively removed via a retro Michael reaction by adding a nucleophilic reducing agent. Therefore, 250mM TCEP were added per 13µL VHH for 10 min at 37°C. This led to a VHH polypeptide containing a single ETAC-Biotin label with a MW of 16240 Da as confirmed by mass spectrometry (Figure 7b). After the TCEP step, this yields to the final and desired product.

### 2.3 The use of ETAC-biotin-linker labeled CD56-specific VHHs in flow cytometry

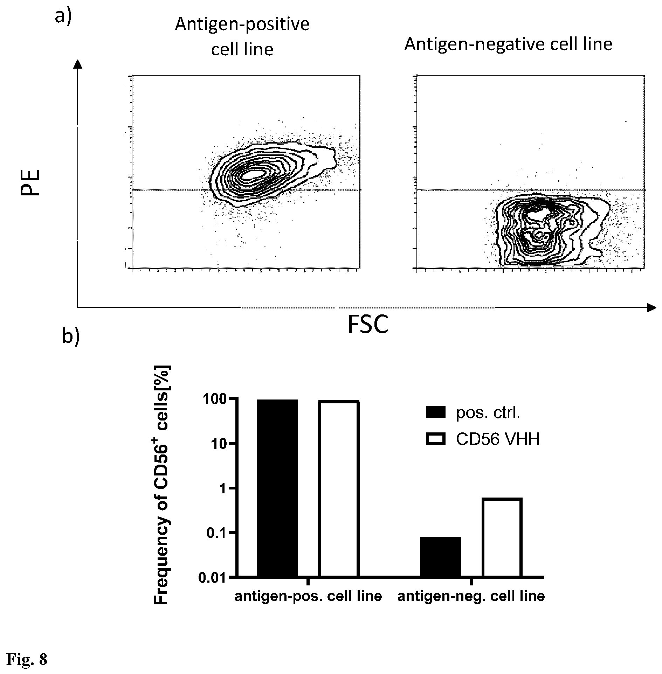

To show the application of ETAC-biotin labeled CD56 VHHs for flow cytometry, CD56-specific VHH molecules were used to detect tumor cells that are known to express CD56. To show the specific binding of ETAC-biotin labeled CD56 specific VHHs, 5µg/mL of the conjugated VHH were incubated with CD56 positive tumor cells and CD56 negative cells for 1h at 4°C. The cell suspension was washed with PEB. Binding of ETAC-biotin labeled CD56 specific VHHs was detected via secondary staining with a PE labeled biotin-specific secondary antibody (REA746).A CD56 specific IgG1 (REA196-PE) was used as a positive control. Next, the cell suspension was washed with PEB. Signal intensity of PE positive tumor cells was analyzed via flow cytometry on antigen positive cells and on antigen negative cells. Frequency of CD56 positive cells was plotted for antigen positive and antigen negative cells after incubation with either ETAC-biotin labeled CD56 specific VHHs or a CD56 specific IgG1 (REA196-PE) as positive control. Specific binding of ETAC-biotin labeled VHHs was detected (Figure 8a). The CD56 ETAC-biotin labeled VHH staining pattern was comparable to the positive control. No binding of the ETAC-biotin labeled CD56 VHH was detected via flow cytometry on antigen negative cells (Figure 8b).

## Claims

1. Method for providing a polypetide with a detectable label, wherein the polypetide comprises at least 5 amino acids of which a first amino acid is C (Cysteine) and at least one second amino acid is selected from the group consisting of S (Serine), T (Threonine), Y (Tyrosine), K (Lysine), H (Histidine) and R (Arginine) **characterized by** the steps
a) initiating Michael addition between a first and a second amino acid by providing a reagent according to formula (I) 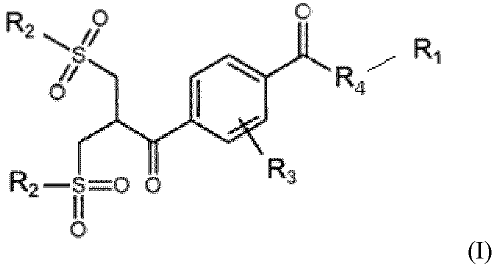 With
R1 = -Biotin, Thiamine, Peptide with 2 - 30 amino acids, oligonucleotides with 6 - 100 nucleotides, -DBCO, DBCO, -N3,N3, -Fluorophore, -Fluorescent protein
R2 = substituted or unsubstituted aromatic residues
R3 = H, F, NO2, alkyl with 1 to 5 carbon atoms
R4 = direct bond, substituted or unsubstituted alkyl, amine or amide residue with 1 to 20 carbon atoms
b) Adding a nucleophilic agent to remove the Michael addition products between two second amino acid by retro-Michael addition.

2. Method according to claim 1 **characterized in that** at least one second amino acid is provided at the C-terminus of the polypeptide.

3. Method according to claim 1 or 2 **characterized in that** the nucleophilic agent is selected from the group consisting of Dithiothreitol (DTT), Tris(2-carboxyethyl)phosphine (TCEP), Triphenylphosphine (PPh3), Hydrazine (N2H4), Tetrakis(dimethylamino)ethylene (TDAE), Mercaptoethanol, , Formic acid, Trichlorosilane (HSiCl3), Phosphine (PH3), Dimethyl sulfide borane (Me2S•BH3), Thioacetic acid, Tris(trimethylsilyl)phosphine, Tri-n-hexylphosphine,, Ammonia (NH3), Cysteine, Selenocysteine, Thiophenole, Glutathione (GSH), Iodide, Methylamine, Hydrogen sulfide

4. Method according to any of claims 1 to 3 **characterized in that** the reagent has formula (II) 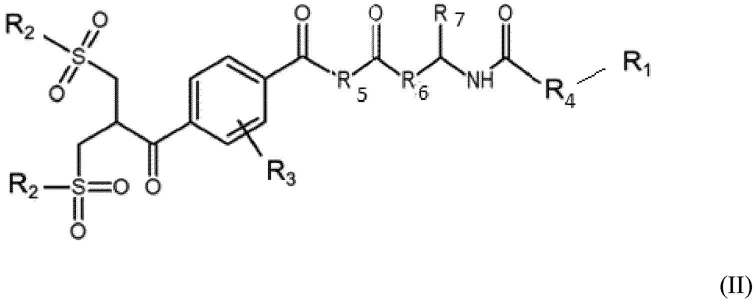 With
R1 = -Biotin, Thiamine, Peptide with 2 - 30 amino acids, oligonucleotides with 6 - 100 nucleotides, -DBCO, DBCO, -N3,N3, -Fluorophore
R2 = substituted or unsubstituted aromatic residues
R3 = H, F, NO2, alkyl with 1 to 5 carbon atoms
R4 = direct bond, substituted or unsubstituted alkyl, amin or amid residue with 1 to 20 carbon atoms
R5 = direct bond, substituted or unsubstituted alkyl or amin residue with 1 to 20 carbon atoms
R6 = direct bond, substituted or unsubstituted alkyl or amin residue with 1 to 20 carbon atoms
R7: substituted or unsubstituted alkyl, carboxy, amin or hydroxy residue with 1 to 20 carbon atoms

5. Method according to any of claims 1 to 4 **characterized in that** the reagent has formula (III) 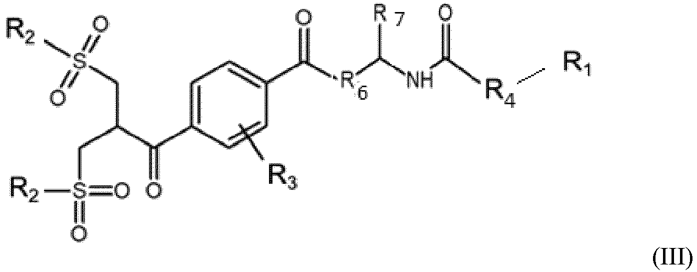 With
R1 = -Biotin, Thiamine, Peptide with 2 - 30 amino acids, oligonucleotides with 6 - 100 nucleotides, -DBCO, DBCO, -N3,N3, -Fluorophore
R2 = substituted or unsubstituted aromatic residues
R3 = H, F, NO2, alkyl with 1 to 5 carbon atoms
R4 = direct bond, substituted or unsubstituted alkyl, amin or amid residue with 1 to 20 carbon atoms
R6 = direct bond, substituted or unsubstituted alkyl or amin residue with 1 to 20 carbon atoms
R7: substituted or unsubstituted alkyl, carboxy, amin or hydroxy residue with 1 to 20 carbon atoms

6. Method according to any of claims 1 to 5 **characterized in** the polypetide is provided with 2 to 10 Histidine units (H) at the C terminal .

7. Method according to any of claims 1 to 6 **characterized in that** the polypetide is a single-domain antibody (VHH), an antibody fragment or a full size antibody.

8. Use of the method according to any of claims 1 to 7 to provide polypetides with in CAR-Adapter molecules, in Cell staining reagents, in cell separation/ labeling/ enrichment/ depletion processes, in protein purification and for Cell activation with a detectable label.

## Patentansprüche

1. Verfahren zur Bereitstellung eines Polypeptids mit einer nachweisbaren Markierung, wobei das Polypeptid mindestens 5 Aminosäuren umfasst, von denen eine erste Aminosäure C (Cystein) ist und mindestens eine zweite Aminosäure ausgewählt ist aus der Gruppe bestehend aus S (Serin), T (Threonin), Y (Tyrosin), K (Lysin), H (Histidin) und R (Arginin), das durch die folgenden Schritte gekennzeichnet ist:
a) Einleiten einer Michael-Addition zwischen einer ersten und einer zweiten Aminosäure durch Bereitstellen eines Reagenzes gemäß Formel (I) 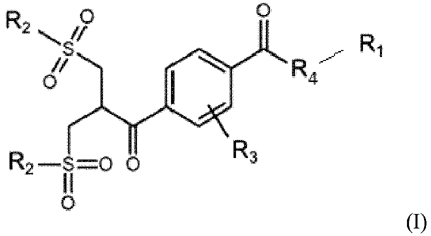 mit
R1 = -Biotin, Thiamin, Peptid mit 2-30 Aminosäuren, Oligonukleotide mit 6-100 Nukleotiden, -DBCO, DBCO, -N3,N3, -Fluorophor, -Fluoreszierendes Protein
R2 = substituierte oder unsubstituierte aromatische Reste
R3 = H, F, NO2, Alkyl mit 1 bis 5 Kohlenstoffatomen
R4 = direkte Bindung, substituierter oder unsubstituierter Alkyl-, Amin- oder Amidrest mit 1 bis 20 Kohlenstoffatomen
b) Zugeben eines nucleophilen Mittels, um die Michael-Additionsprodukte zwischen zwei zweiten Aminosäuren durch Retro-Michael-Addition zu entfernen.

2. Verfahren nach Anspruch 1, **dadurch gekennzeichnet, dass** mindestens eine zweite Aminosäure am C-Terminus des Polypeptids bereitgestellt wird.

3. Verfahren nach Anspruch 1 oder 2, **dadurch gekennzeichnet, dass** das nucleophile Mittel ausgewählt ist aus der Gruppe bestehend aus Dithiothreitol (DTT), Tris(2-carboxyethyl)phosphin (TCEP), Triphenylphosphin (PPh3), Hydrazin (N2H4), Tetrakis(dimethylamino)ethylen (TDAE), Mercaptoethanol, Ameisensäure, Trichlorsilan (HSiCl3), Phosphin (PH3), Dimethylsulfidboran (Me2S•BH3), Thioessigsäure, Tris(trimethylsilyl)phosphin, Tri-n-hexylphosphin, Ammoniak (NH3), Cystein, Selenocystein, Thiophenol, Glutathion (GSH), Iodid, Methylamin, Schwefelwasserstoff.

4. Verfahren nach einem der Ansprüche 1 bis 3, **dadurch gekennzeichnet, dass** das Reagenz die Formel (II) aufweist: 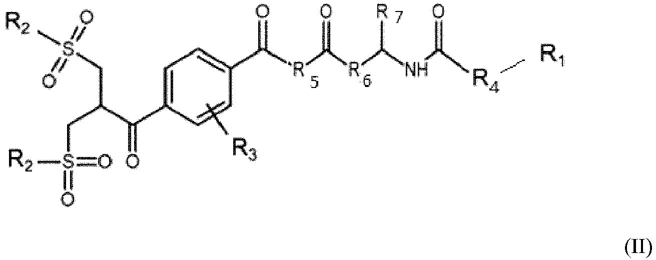 mit
R1 = -Biotin, Thiamin, Peptid mit 230 Aminosäuren, Oligonukleotide mit 6-100 Nukleotiden, -DBCO, DBCO, -N3,N3, -Fluorophor,
R2 = substituierte oder unsubstituierte aromatische Reste
R3 = H, F, NO2, Alkyl mit 1 bis 5 Kohlenstoffatomen
R4 = direkte Bindung, substituierter oder unsubstituierter Alkyl-, Amin- oder Amidrest mit 1 bis 20 Kohlenstoffatomen
R5 = direkte Bindung, substituierter oder unsubstituierter Alkyl- oder Aminrest mit 1 bis 20 Kohlenstoffatomen
R6 = direkte Bindung, substituierter oder unsubstituierter Alkyl- oder Aminrest mit 1 bis 20 Kohlenstoffatomen
R7: substituierter oder unsubstituierter Alkyl-, Carboxy-, Amin- oder Hydroxyrest mit 1 bis 20 Kohlenstoffatomen.

5. Verfahren nach einem der Ansprüche 1 bis 4, **dadurch gekennzeichnet, dass** das Reagenz die Formel (III) aufweist: 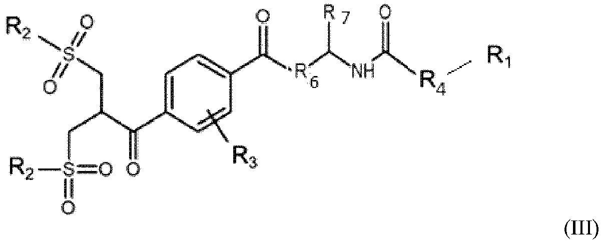 mit
R1 = -Biotin, Thiamin, Peptid mit 230 Aminosäuren, Oligonukleotide mit 6-100 Nukleotiden, -DBCO, DBCO, -N3,N3, -Fluorophor,
R2 = substituierte oder unsubstituierte aromatische Reste
R3 = H, F, NO2, Alkyl mit 1 bis 5 Kohlenstoffatomen
R4 = direkte Bindung, substituierter oder unsubstituierter Alkyl-, Amin- oder Amidrest mit 1 bis 20 Kohlenstoffatomen
R6 = direkte Bindung, substituierter oder unsubstituierter Alkyl- oder Aminrest mit 1 bis 20 Kohlenstoffatomen
R7: substituierter oder unsubstituierter Alkyl-, Carboxy-, Amin- oder Hydroxyrest mit 1 bis 20 Kohlenstoffatomen.

6. Verfahren nach einem der Ansprüche 1 bis 5, **dadurch gekennzeichnet, dass** das Polypeptid am C-Terminus mit 2 bis 10 Histidineinheiten (H) bereitgestellt wird.

7. Verfahren nach einem der Ansprüche 1 bis 6, **dadurch gekennzeichnet, dass** das Polypeptid ein Einzeldomänen-Antikörper (VHH), ein Antikörperfragment oder ein Volllängenantikörper ist.

8. Verwendung des Verfahrens nach einem der Ansprüche 1 bis 7 zur Bereitstellung von Polypeptiden mit CAR-Adaptermolekülen, in Zellfärbereagenzien, in Zellseparations-/Markierungs-/Anreicherungs-/ Depletionsprozessen, in der Proteinreinigung und zur Zellaktivierung mit einer nachweisbaren Markierung.

## Revendications

1. Procédé de fourniture d'un polypeptide avec un marqueur détectable, dans lequel le polypeptide comprend au moins 5 acides aminés dont un premier acide aminé est C (cystéine) et au moins un second acide aminé est choisi dans le groupe constitué par S (sérine), T (thréonine), Y (tyrosine), K (lysine), H (istidine) et R (arginine) **caractérisé par** les étapes
a) initiation d'une addition de Michael entre un premier et un second acide aminé en fournissant un réactif selon la formule (1) 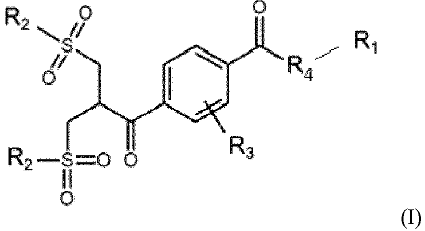 avec
R1 = -biotine, thiamine, peptide avec 2 à 30 acides aminés, oligonucléotides avec 6 à 100 nucléotides, -DBCO, DBCO, -N3,N3, -fluorophore, -protéine fluorescente
R2 = résidus aromatiques substitués ou non substitués
R3 = H, F, NO2, alkyle avec 1 à 5 atomes de carbone
R4 = liaison directe, alkyle substitué ou non substitué, résidu d'amine ou d'amide avec 1 à 20 atomes de carbone
b) addition d'un agent nucléophile pour enlever les produits d'addition de Michael entre deux seconds acides aminés par addition de rétro-Michael.

2. Procédé selon la revendication 1, **caractérisé en ce que** au moins un second acide aminé est fourni à l'extrémité C-terminale du polypeptide.

3. Procédé selon la revendication 1 ou 2, **caractérisé en ce que** l'agent nucléophile est choisi dans le groupe constitué par le dithiothréitol (DTT), la tris(2-carboxyéthyl)phosphine (TCEP), la triphénylphosphine (Pph3), l'hydrazine (N2H4), le tétrakis(diméthylamino)éthylène (TDAE), le mercpatoéthanol, l'acide formique, le trichlorosilane (HsiCl3), la phosphine (PH3), le sulfure de diméthyle borane (Me2S•BH3), l'acide thioacétique, la tris(triméthylsilyl)phosphine, la tri-n-hexylphosphine, l'ammoniaque (NH3), la cystéine, la sélénocystéine, le thiophénole, la glutathione (GSH), l'iodure, la méthylamine, le sulfure d'hydrogène.

4. Procédé selon l'une quelconque des revendications 1 à 3, **caractérisé en ce que** le réactif a la formule (II) 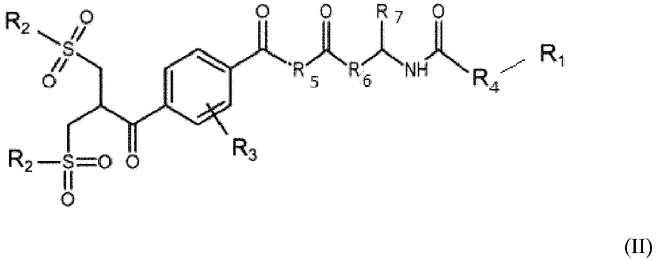 avec
R1 = -biotine, thiamine, peptide de 2 à 30 acides aminés, oligonucléotides avec 6 à 100 nucléotides, -DBCO, DBCO, -N3,N3, -fluorophore
R2 = résidus aromatiques substitués ou non substitués
R3 = H, F, NO2, alkyle avec 1 à 5 atomes de carbone
R4 = liaison directe, alkyle substitué ou non substitué, résidu d'amine ou d'amide avec 1 à 20 atomes de carbone
R5 = liaison directe, alkyle substitué ou non substitué ou résidu d'amine avec 1 à 20 atomes de carbone
R6 = liaison directe, alkyle substitué ou non substitué ou résidu d'amine avec 1 à 20 atomes de carbone
R7 : liaison directe, alkyle substitué ou non substitué, carboxy, amine ou résidu d'hydroxy avec 1 à 20 atomes de carbone.

5. Procédé selon l'une quelconque des revendications 1 à 4, **caractérisé en ce que** le réactif a la formule (III) 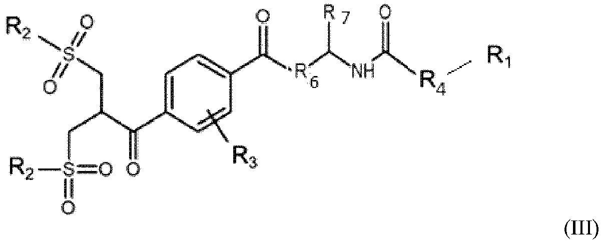 avec
R1 = -biotine, thiamine, peptide de 2 à 30 acides aminés, oligonucléotides avec 6 à 100 nucléotides, -DBCO, DBCO, -N3,N3, -fluorophore
R2 = résidus aromatiques substitués ou non substitués
R3 = H, F, NO2, alkyle avec 1 à 5 atomes de carbone
R4 = liaison directe, alkyle substitué ou non substitué, résidu d'amine ou d'amide avec 1 à 20 atomes de carbone
R5 = liaison directe, alkyle substitué ou non substitué ou résidu d'amine avec 1 à 20 atomes de carbone
R6 = liaison directe, alkyle substitué ou non substitué ou résidu d'amine avec 1 à 20 atomes de carbone
R7 : liaison directe, alkyle substitué ou non substitué, carboxy, amine ou résidu d'hydroxy avec 1 à 20 atomes de carbone.

6. Procédé selon l'une quelconque des revendications 1 à 5, **caractérisé en ce que** le polypeptide est fourni avec 2 à 10 unités d'histidine (H) à l'extrémité C-terminale.

7. Procédé selon l'une quelconque des revendications 1 à 6, **caractérisé en ce que** le polypeptide est un anticorps à simple domaine (VHH), un fragment d'anticorps ou un anticorps de taille complète.

8. Utilisation du procédé selon l'une quelconque des revendications 1 à 7 pour fournir des polypeptides avec des molécules adaptatrices de CAR dans des réactifs de coloration de cellule dans des processus de déplétion enrichissement de marquage de séparation de cellules, dans la purification de protéines et pour l'activation de cellules avec un marqueur détectable.
